# EUROPEAN PATENT APPLICATION

(11) **EP 4 618 092 A1**
(43) Date of publication of application: **17.09.2025**
(21) Application number: 24162599.5
(22) Date of filing: 11.03.2024
(51) Int. Cl.: G16H 20/40, A61M 1/14, G16H 40/63

(54) **METHOD FOR CONTROLLING AN AUGMENTED REALITY DEVICE, AUGMENTED REALITY SYSTEM AND MEDICAL SYSTEM**

(71) Applicant: Fresenius Medical Care Deutschland GmbH, 61352 Bad Homburg (DE)
(72) Inventor: Kipp, Sabine, 61352 Bad Homburg (DE); Heinze, Marcel, 61352 Bad Homburg (DE); Witzel, Stephan, 61352 Bad Homburg (DE)
(74) Representative: Schwarz, Claudia

(57) **Abstract**

The present invention relates a system for an automated generation and provision of assistance when operating a medical device such as a dialysis device. In particular, the present invention provides an automated computation of a virtual assistant in form of a person or character. The virtual assistant is provided to the user by means of an augmented reality device.

## Description

The present invention relates to a method for controlling an augmented reality device and in particular to a method for controlling an augmented reality device used for operating a dialysis device. The present invention further relates to an augmented reality system, in particular an augmented reality system for use in a medical environment comprising a dialysis a device. Further, the invention relates to a medical system comprising such an augmented reality system.

Even though the present invention is described in the following by way of example with reference to a dialysis device, the general concept may be also applicable to other devices, in particular other medical devices, especially blood treatment devices.

More and more medical devices are becoming increasingly complex. An example of such a complex medical device is a dialysis device. Such medical devices require complex operations and configurations which have to be performed by the user. Further, numerous accessories and consumables should to be taken into account when operating medical devices.

Since improper operations could be dangerous for a patient, it is of great importance that a user, e.g. a nurse or doctor, is familiar with the device. In case of uncertainties, it is desirable that the user can be guided fast, safely and reliably through the tasks.

At present, the user has to read the manual of the respective device in order to learn to operate it. However, even if the user has some experience with the use of such a device, there may be still some situations the user is less familiar with. In such situations, the user might have to refer to a manual or to contact another person or trainer. Such additional operations may require additional time which can be problematic in time-critical condition. Further to this, and another person or trainer may be not available in time.

In view of this, there is a need for improved assistance of the user when operating a complex medical device such as a dialysis device.

### Summary

The present invention provides a method for controlling an augmented reality device, an augmented reality system and a medical system with the features of the independent claims. Further advantageous embodiments are subject-matter of the dependent claims.

According to a first aspect, the present invention provides a method for controlling an augmented reality, AR, device. The AR device may be used for operating a dialysis device. The method further comprises a step of computing an assistance scheme. The assistance scheme may be an assistance scheme for carrying out a task to be executed at least in part by the user, operating the dialysis device. The task may be a task to be performed directly on the dialysis device or indirectly, e.g. on peripheral parts or accessories, like e.g. consumables, thereof. The computation of the assistance scheme is based on the received input data from the dialysis device. The method further comprises a step of generating or providing an output on an output component of the AR device, wherein the output component needs not necessarily implemented fully on the AR device (it might also at least in part be implemented on another device). The output on the output component of the AR device is generated or computed by applying the computed assistance scheme. The generated output comprises a representation of a virtual character or person. Such a representation of a virtual character or person may be considered as a so-called avatar. Accordingly, the user is assisted in operating the dialysis device by technical means.

The first aspect relates to the claimed method. Features, advantages or alternative embodiments which are described or claimed with respect to the method can be also transferred or assigned to the other claimed objects (e.g. the system, the augmented reality device, computer program) and vice versa. In other words, the device or system can be improved with features described or claimed in the context of the method and vice versa. In this case, the functional features of the method are embodied by structural units of the apparatus or device or system and vice versa, respectively. Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the claimed device or apparatus claim is adapted to carry out the claimed method.

According to a further aspect the present invention provides an Augmented Reality, AR, system for use in a medical environment. In particular, the medical system may comprise a dialysis device. The AR system comprises an AR device and a control device. The control device may be but needs not necessarily be configured to receive input data, in particular to receive input data from the dialysis device. The control device is configured to execute the method described above. The control device is further configured to compute an assistance scheme, in particular an assistance scheme for carrying out a task to be executed in connection with or by operating the dialysis device. The assistance scheme may be computed based on the received input data from the dialysis device. The control device is further configured to generate or provide an output, in particular to generate the output on an output component of the AR device. The output on the output component of the AR device is generated by applying the computed assistance scheme. The generated output comprises a representation of a virtual character or person.

The AR device may comprise a sensor component. Alternatively or in addition, the sensor component may at least in part be implemented on an AR-external device, like the dialysis device and/or a mobile device. It is also possible, that the assistance scheme provides output data, instructing to output the generated output (e.g. the avatar) on more than one output component, e.g., on the AR device and on the dialysis device and/or another device.

According to still further aspect, the present invention provides a medical system comprising a dialysis device and an AR system according to the previous aspect.

In still a further aspect, the present invention provides a computer program for operating the control device for the AR system. The method may be provided as computer program and/or the method may be computer-implemented.

The method according to one aspect, mentioned above, may comprise using a large language model, LLM. In particular, the LLM may be used to generate the output, e.g., in the form of calculating actuations of an avatar. The LLM may be trained for acquiring the abilities, mentioned before by learning statistical relationships from text documents or other information sources or files during a computationally intensive self-supervised and semi-supervised training process. The LLM may be built with a decoder-only transformer-based architecture.

Alternatively or in addition other architectures may be used, such as recurrent neural network variants and Mamba (a state space model). In particular, the LLM may be used, to get an answer to a question provided in relation to the dialysis device. The answer may be provided on the output component of the AR device or on an output component of the dialysis device or of a user's (e.g., mobile) device. The user can ask the system by using the LLM, for example, which part of the machine is currently in front of him, where to place a disposable, where a certain part of the machine (e.g. the blood pump) is located, or ask for help with the next step. The system recognises the situation in the room with the help of AR sensors and/or the at least sensor component, evaluates the user's verbal interaction of the user accordingly using the LLM and provides corresponding information such as AR highlighting, text, arrows and/or prepared videos and/or graphics to answer the verbal user enquiry. This can be done in any language if the LLM has the appropriate language capabilities. The system may respond automatically in the language of the enquiry.

The invention (method, system and the other claimed subject matters) may be provided in two embodiments.

In a first embodiment, the method is triggered or initiated by a trigger command of the dialysis device. In this case the method may comprise the step of receiving input data. In particular, the input data may comprise the trigger command, to be received from the dialysis device. The computed assistance scheme may be based on the received input data. In this embodiment, the dialysis device issues the trigger command, which may comprise requesting a particular operation to be performed by the user.

In a second embodiment, the method is triggered by a trigger command, which may be received via the AR device. The trigger command may request the user to perform a task with regard to the dialysis machine.

Alternatively or in addition, the method may be triggered on request of the user himself. In particular, the user may input a request for assistance. The input may be performed via gesture and/or voice command and/or input on a user interface of the dialysis device and/or an input component on the AR device. For example, for this purpose, the AR device may be configured with an assistance trigger button. In case this assistance trigger button is activated, a message is sent from the AR device to the controller to initiate execution of the method for providing assistance to the user.

An assistance scheme defines a scheme or pattern for providing the assistance. An assistance scheme may define how, where (in particular, where or on which device the output component is implemented and instructed for providing the indications) and/or in which form the assistance in form of the generated output is to be provided technically, in particular with respect to the determination of the appropriate output component, time intervals, duration, output format (optical, acoustical, and/or haptical etc.), and/or intensity (brightness for optical indications or volume for acoustical indications etc). The assistance scheme may comprise or refer to optical, acoustic and/or haptic output. For example, the assistance scheme may refer to or comprise haptic indications such as a shaking or vibration in order to draw the user's attention. The assistance scheme may refer to or comprise acoustic indications such as a beep, warning tone, melody, any other kind of sound or even a spoken text message, which may be provided by the representation of the virtual character or person. The assistance scheme may further refer to or comprise optical indications such as arrows, lines, circles, boxes, icons, images, text messages, video sequences, etc., in order to guide a user through the related task. The indications may be provided on the output component of the AR device. However, it is understood, that any other kind of assistance scheme may be used in order to provide appropriate assistance to a user for carrying out the desired task.

Alternatively or in addition, the assistance scheme may define the kind, form, and/or manner, how the representation of the virtual character or person may be generated and provided to the user. The assistance scheme may define how long and/or how often the representation is provided, whether or not additional elements are provided as output in addition to the representation, for example, acoustic or haptic output or output in another form. Alternatively or in addition, the assistance scheme may define properties of the representation of the virtual character or person such as a static or a dynamic appearance, and/or a spatial position where the representation of the virtual character person is provided, any kind of content which is provided to the user by the representation of the virtual character or person, any kind of content or information which is provided to the user by the representation of the virtual character or person, and/or a time period (how long) for providing the representation of the virtual character or person, etc. The assistance scheme further comprises information to guide a user when operating the medical device. For example, the assistance scheme may specify consumables which have to be filled in the medical device, accessories which have to be attached to the medical device, operational steps how a desired task has to be carried out, etc.

A task to be executed in the context of the present invention may be any kind of operation or action in connection with the related medical device. A task may require at least one activity and in particular a set or sequence of activities to be carried out manually by the user or automatically via instruction.

The present invention is based on the finding that operating a complex medical device such a dialysis device is a great demand for users. A user has to study the manuals and has to be extensively trained to use such a device. In particular, there may be numerous exceptional situations which have to be handled by the user. Since some failures of operating the device may lead to a health risk, the user has to operate to device very carefully in order to avoid such failures. This may be a real challenge, especially under time pressure.

The present invention therefore takes into account this finding and aims to provide additional assistance to the user by means of an AR device. In particular, the present invention aims to assist the user operating complex medical devices by providing additional assistance in form of automatically generating situation-based assistance for a user and providing this generated assistance by means of an AR device.

An AR device in the context of the present invention may be understood any as kind of device providing AR features. For example, such an AR device may be an AR or mixed reality, XR, headset such as, e.g., Microsoft HoloLense. However, any other kind of AR or XR device may be possible, too. The AR device may be also any other kind of device providing AR features. For example, a smart phone, tablet computer or any other kind of device with a camera and a display may be used as an AR device. In general, any kind of device which has capabilities for providing an overlay of a real scene with additional (artificial) elements may be used as AR device. "Augmented" in the present context refers to the fact, that the real or physical scene may be augmented or enriched with additional information, which may be provided as virtual indications.

An output component in the context of the present invention may be any kind of component for outputting the generated output, e.g., in the form of optical, haptic and/or acoustic indications. The output component may be implemented, at least in part, by the above-mentioned AR device. In particular, the output component may be part of the AR device. For example, the output component may comprise a display for providing optical information or indications. In particular, the output component may comprise a display device for overlaying a real scene with additional virtual elements/indications. For this purpose, a semi-transparent display may be used for providing additional indications on a real scene to which the display is pointed. Alternatively, a scene may be captured by a camera, and the image data from the camera may be overlaid with additional virtual indications on the display. Alternatively, the output component may be implemented, at least in part, as a separate device to the AR device. The output component may comprise different output component parts, e.g., a display of the AR device, a loudspeaker of the AR device or on another device, an output component of the medical (e.g., dialysis) device. Several of these output component parts may be used in parallel. Alternatively or in addition, the output component may be implemented on a device, which is external to the AR device, like e.g., the dialyses device itself or another device, like a mobile device. Thus, alternatively or in addition the output component may be implemented on another device, although it is referred to as "output component of the AR device". In the latter case the output component may be outsourced or transferred from the AR device to an AR-external device.

The representation of the virtual character or person in the context of the present invention may be considered as any kind of output which may be noticed by the user as a character or person. For example, such a representation of the virtual character a person may be a digital avatar or the like. The representation of the virtual character or person may be provided in form of an optical representation, e.g. a computer-generated representation of a person, or an abstract representation of a character. In particular, such an optical representation of a virtual character may be also a cartoon character or even an (animated) symbol such as a paperclip or a star. For short, any appropriate realistic or abstract representation which is appropriate for providing the desired information to the user may be used as a virtual character or person. The representation of the virtual character or person may comprise actuated limbs. This has the effect that the provided representation may be actuated according to target movement of the user for executing the task. With this, it is possible to show the user how to move and/or where to position and/or how to pose in order to execute the task correctly. Thus, it is possible to transfer positional and/or pose and/or movement data via the output component to the AR device.

The output of a virtual character or person is not only limited to graphical or optical output. Moreover, the output may also comprise, for example, acoustic output such as spoken text, sound signals or the like. For example, a virtual character or person may be represented by a voice which speaks to the user. Further to this, any other way for providing information to the user, e.g. by means of haptic output or the like, may be possible, too.

A task to be executed in the context of the present invention may be any kind of operation in connection with the related medical device. Such a task may be, for example, preparing the medical device for a desired task/job (e.g. setup of the medical device, in particular dialysis machine, especially prior to commissioning of the medical device). For this purpose, a user may have to mount one or more attachments or accessories on the medical device, insert one or more consumables, make specific settings on the medical device or perform any other required operation. If necessary, particular data of a patient have to be taken into account. A task may be also, for example, monitoring the medical devices during operation. For this purpose, the user may have to monitor the status messages of the medical device, monitor a filling level of the consumables, monitor flow rates, etc. In case that one of the parameters is out of tolerances, the user has to react on this in order to ensure safe operation. In another example, a task may be, for example, reacting in case of a failure or error message. For example, if the medical device detects a failure or problem, the user has to take the necessary steps in order to remove the reason for such a problem. However, it is understood, that a task may comprise any other kind of operation in connection with the related medical device, such as maintenance, normal operation, testing, and/or training.

A task may be specified or selected, for example, by the user, for instance by user input. Additionally or alternatively, a task may be determined fully or at least partly automatically by means of executing a task function. For this purpose, further information such as data from the medical device, from a patient database and/or other sources/databases may be taken into account in order to determine a desired task by the task function. The task function may be configured to assist and/or train the user on certain tasks which may still be improved. The task function may be user-specific. With this feature, the assistance may be adapted specifically for the user. Further to this, it may be also possible that another person, for example a trainer or instructor, may select a task which should be executed by the user.

In a possible embodiment, the assistance scheme is computed by applying a machine learning approach or tool. The machine learning tool may be denoted as artificial intelligence and may be implemented as neural network architecture, in particular as a DNN, deep neural network. The neural network may be trained with training data. The training data may comprise the task to be executed and an output for providing assistance. Alternatively, the training data may comprise an operational setting of the medical device and/or monitored activities of the user and/or a skill level of the user. The neural network is trained to provide an output according to an assistance scheme for new, unseen tasks, and/or operational settings and/or monitored activities of the user. The training may be supervised or unsupervised or semi-supervised.

In a possible embodiment, the generated output comprises optical, acoustic and/or haptic output, which may be output on the output component of the AR device. For example, optical output may comprise a - in particular optical -representation of a virtual character or person such as an avatar.

The representation may be a static representation. Such a static representation may be, for example, a (computer-generated) illustration of a person or character for indicating a desired operation which shall be performed by the user. For example, the representation may illustrate a person which is operating a control element of the medical device. If appropriate, additional text may be also provided in addition to the representation of the virtual person or character.

The representation may be also a dynamic representation. For example, the representation may be, for example, a (computer-animated) person or character. Such an animated person or character may be used to demonstrate a sequence of positions and/or poses or process or movement which shall be operated by the user. For this purpose, the representation may be calculated by means of a representation algorithm. The representation algorithm is configured to actuate the limbs of the person or character in order to mimic the correct movement of the user for executing the task. For example, the dynamic representation may be provided such that the virtual person or character simulates or mimics the position, sequence of poses, and/or movement of a user when executing the respective operation on the medical device correctly. The representation algorithm calculates limb actuations such as the limb actuations represent or indicate a movement that is required to operate the dialyses device, in particular the task to be carried out.

In a possible embodiment the computed assistance scheme may comprises rules for representation of limb actuations of the virtual character or person. If, for example, the virtual character or person relates to a computer-generated and/or computer-animated person or character, or at least part of the whole person or character (e.g. an arm with a hand, or a hand with fingers), the assistance scheme may specify rules how the individual limbs of the provided person or character shall be modelled in order to represent the desired movement as assistance to guide the user. For example, the specified rules may describe the movement of the fingers for operating a control element of the medical device, or the movement of an arm with a hand in order to illustrate the attachment of an accessory. The level of detail of the calculated representation may be adapted with respect to the task. For example, if the task requires gross motor skills, then a coarse representation with less level of detail is calculated, whereas if the task requires fine gross motor skills, a more detailed representation with higher level of detail is calculated.

In a possible embodiment, the generated output comprises providing a desired movement by the simulation of limb actuations of the virtual character or person. For example, the representation of the virtual person or character may be generated based on a computer-implemented approach or a computer-animated processing of the character or person. Especially, specifications and/or rules according to the computed assistance scheme for limb actuations may be taken into account in order to generate the virtual character or person. In this way, a realistic illustration of the virtual character or person can be obtained which makes it easy to the user to understand the desired operations on the medical device.

In a possible embodiment, a level of detail for generating the output is adjustable. This level of detail may be adjusted, for example, manually by an appropriate input of the user. However, as described in more detail below, the level of detail may be also adapted automatically based on appropriate rules. The level of detail may specify, for example, a level of detail how complex the representation of the virtual person or character is provided to the user. However, the level of detail may also specify a level how precisely the individual operations or subtasks for operating the medical device are provided to the user. For example, a very unexperienced user may select a high level of detail so that all or at least a huge number of sub-tasks or operations are provided to the user. A more experienced user may select a lower level of detail so that only a reduced number of sub-tasks or operations are provided to the user. In this way, the unexperienced user is provided with all required information to carry out the desired task, and the more experienced user is provided with less information in order to avoid flooding the user with information or confusing the user.

In a possible embodiment, the method may comprise a step of monitoring activities of the user. In particular, the activities of the user may be monitored for determining a skill level of the user. A skill level may comprise a level of experience of the user. A level of experience may be determined based on historical stored data. Alternatively or in addition, the level of experience may be determined on data, representing the user's activities, performed at the dialysis device. This can be used to indicate, for example, how much experience the user has already gained. The skill level may be derived in any appropriate manner based on the monitored activities of the user, in particular of the actually monitored activities. Based on the determined skill level of the user, the level of detail for generating the output may be adjusted. In this way, an automated approach for adapting the level of detail for assisting the user can be achieved.

In a possible embodiment, the method may comprise a step of receiving data related to a skill level of the user. Additionally or alternatively, preferences of the user may be received. The data related to a skill level of the user and/or the preferences of the user may be received, for example from a database. The database may be a local storage device or remote storage, for example storage in the cloud. Based on the received data, the level of detail for generating the output may be adjusted. Accordingly, the assistance for user can be individually adapted.

In a possible embodiment, the assistance scheme and/or the output may be computed based information received from the AR device. In particular the assistance scheme and/or the output may be computed based on a type of the dialysis device, monitored activities of the user, a user identity, a geographical region identifier and/or a time information. "Time information" refers to time parameters. Time parameters may indicate when or at what time and/or how long (duration) the output is to be provided. Alternatively or in addition, time parameters may indicate the time (e.g., during the course of the day) the assistance is requested. For example, if the assistance is requested or triggered during daytime, the assistance scheme may define to provide the acoustic output with a volume which is adapted to daily surrounding noise, whereas if the assistance is requested or triggered during night time, the assistance scheme may define to provide the acoustic output with a (lower) volume which is adapted to less surrounding noise or silence. The same applies to an optic output, where the intensity is adapted to the time of the day, when the assistance is requested. However, it is understood, that any other appropriate information may be also taken into account for computing the appropriate, assistance scheme, in particular an assistance scheme which fits the current demands.

In a possible embodiment, the method may comprise a step of receiving user input. The user input may be received, for example, by an input component, e.g., a user interface,. Especially, the user input comprises a gesture, a voice input and/or a haptic input. Accordingly, the assistance scheme may be computed based on the received user input. In this way, the user may control and adapt the provided assistance, in particular, the control may be performed even in real time. For example, a level of detail may be adapted according to such a user input.

Furthermore, it may be also possible to switch on or off the provision of assistance based on the virtual person or character. However, any other kind of input may be also taken into account. For example, the user may use such an input in order to specify a desired task or may request any kind of additional information, in particular information in connection with the operation of the medical device.

In a possible embodiment the method further comprises a step of scanning a spatial environment of the dialysis device. The spatial environment may be scanned, for example, by the at least one sensor component. The sensor component may be a sensor component of the AR device and/or may be provided as separate component. Accordingly, the assistance scheme may be computed based on data of the scanned spatial environment. For example, in computing the assistance scheme, it may be possible to take into account the available space in the environment of the medical device. Accordingly, spatial limitations, e.g. limitations with respect to the movement of the user around the medical device or the like, may be taken into account. Furthermore, it may be also possible to identify further objects in the environment, such as available accessories and/or consumables. In this case, the identified further objects may be also taken into account when computing the assistance scheme for the current user.

A sensor component in the context of the present invention may be any kind of input component, in particular any kind of component for sensing an environmental property and/or for receiving user input, e.g., like a user interface, UI, graphical user interface, GUI etc. For example, the sensor component may comprise optical sensor elements such as at least one camera, at least one laser scanner or the like. Alternatively or in addition, the sensor component may also comprise an acoustic sensor element such as a microphone, which can be used, for example, for recording a voice command of the user. Alternatively or in addition, the sensor component may further comprise sensor elements such as a gyroscope, an acceleration sensor (for one or more dimensions) and/or a distance sensor or any other kind of sensor for sensing a position, pose and/or movement of the user. Alternatively or in addition, the sensor element may also comprise one or more communication modules. For example, such a communication module may be a Wl Fl-interface, a Bluetooth-interface, an RFID-reader or any other wireless or wired communication module. However, it is understood, that any other kind of the sensor element may be also comprised the by the sensor component. The sensor component or parts thereof may be part of the augmented reality device. Alternatively or in addition the sensor component may be provided as external device, in particular, external to the medical device and/or external to the augmented reality device. The sensor component may be a part of a mobile device (e.g. smartphone, tablet etc.).

The data, captured by means of the sensor component may relate to 1. the medical device and/or related parts and/or operating conditions thereof and/or to 2. activities of the user, when interacting with or operating the medical device.

In a possible embodiment, the representation of a virtual character or person may be adjustable. In particular, the representation may be adjustable by the user instructions, which may be received via user interface. Especially, the representation of a virtual character or person may be selectable out of a number of prestored templates. The templates may be stored in a local storage or a remote storage device such as a cloud. In this way, the user may adapt the virtual character or person according to his personal preferences. For example, the user may select between one or more male or female persons or characters, adapt a colour of skin, hair, etc. Furthermore, the user may also select, for example, between realistic persons or more artificial characters such as objects, e.g. an animated star or the like.

In the following detailed description of the figures, non-limiting examples of embodiments with their features and further advantages are discussed with reference to the drawing.

### Brief description of the figures

Fig.1: shows a schematic illustration of a medical system according to an embodiment;
Fig. 2: is a schematic illustration of a user with an AR device according to an embodiment;
Fig. 3: depicts a schematic illustration of a user with another AR device according to an embodiment;
Fig. 4: shows a schematic illustration of a flow diagram underlying a method according to an embodiment;
Fig. 5: is a schematic illustration of an AR scene with a virtual person according to an embodiment; and
Fig. 6: is a schematic illustration of an AR scene with a virtual character according to an embodiment.

### Detailed description of the figures

In the following, the invention is described in more detail with reference to embodiments in connection with the figures.

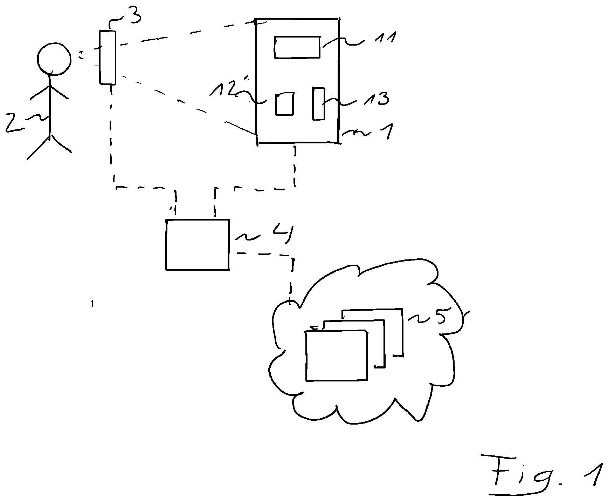

**Figure 1** shows a schematic illustration of a configuration with a medical system according to an embodiment. The medical system comprises a medical device 1, in particular a dialysis device or another blood treatment device. The medical device 1 may be operated by a user 2.

Blood treatment devices, in particular dialysis devices, are medical devices in which the blood of a patient is usually treated extracorporeally. For example, drugs may be added to the blood or blood components removed from it, blood may be heated or cooled. These processes are often monitored by sensors. Blood treatment devices have a variety of means for these purposes, such as pumps, valves, sensors, clamps or the like. Furthermore, medical disposables are often used for blood treatment. Such disposable articles are usually discarded after treatment for reasons of hygiene. Medical disposables, or so-called disposables, are, for example, tube sets for administering medical fluids such as blood or medicaments, filters, such as adsorber filters or dialysis filters, sachets for storing medicaments, and the like. These disposables are usually inserted by a user in the blood treatment device, or connected to the same and removed after treatment from the blood treatment device or separated therefrom.

In the example of figure 1, a medical device 1 (in short: device) is illustrated, wherein the device 1 comprises an input/output component 11, and at least one accessory component 12 representing any kind of attachment, accessory or the like, and a tank component 13 for receiving (liquid) consumables.

The high complexity of such medical devices 1 is a considerable challenge for the users of such a device. Thus, the user 2 should be properly trained before use. Further, it may be desirable to monitor a user 2, when operating the device, in order to recognise a potential improper operation at an early stage. In addition, support to guide the user 2 during operation is also very beneficial.

In the medical system as shown in figure 1, an augmented reality (AR) device 3 may be used for assisting, monitoring and/or training the user 2 in connection with an operation of the medical device 1. Any kind of appropriate device which could be used for AR purposes may be used. For example, the AR device may be any kind of AR headset or AR glasses. Furthermore, any other kind of device such as a tablet computer, a smart phone, etc. which can provide AR capabilities may be possible, too.

The AR device 3 may comprise or may be in - in particular wireless - data connection with one or more output components. For example, an output component may provide optical/graphical output. Such optic/graphical output may be provided, for example on a display. The display may be a non-transparent display on which an image of the environment, in particular an image of the medical device 1 or at least part of the medical device 1, is overlaid with further graphical elements. The display may also be a semi-transparent display so that the user 2 can look through this display in order to recognize the real environment. Furthermore, additional graphical elements may be provided by the semi-transparent display so that graphical indications on the semi-transparent display may provide assistance to the user 2 during operation of the medical device 1.

The AR device 3 may be in a - in particular wireless - data connection to the dialysis device.

The AR device 3 may also comprise one or more further output components such as a loudspeaker, headphones or another device for outputting signals, in particular e.g. acoustic signals. In this way, acoustic signals such as a sound or melody or a spoken text message may be provided to the user 2. Further to this, any other kind of appropriate output component may be also comprised by or connected to the AR device 3. For example, the AR device 3 may also comprise or be connected to an output component for providing haptic signals such as a vibration, shaking or the like.

The AR device 3 may further comprise one or more sensor components for sensing the environment and/or operations of the user 3. For example, the AR device we may comprise one or more image sensing elements such as a camera or the like for capturing image data. The AR device 3 may also comprise any other kind of sensor components, for example a gyroscope, acceleration sensors, velocity sensors, etc. for sensing a movement of the user 2. However, any other kind of appropriate sensing component may be possible, too. For example, a sensor component may also be a scanner, in particular a scanner for reading a QR code or barcode, an RFID reader, etc. Alternatively or in addition, the one or more sensor components may be provided separately form the AR device 3, e.g. as room sensors, or sensors of mobile devices or other sensor components, which are in data connection to the AR device 3.

Preferably, are or at least some of the output components and/or sensor components can be implemented in the AR device 3. However, as mentioned above, it may be also possible to use external output components and/or input components which are not implemented in the AR device 3, but which are in data exchange with the same.

When using the medical device 1, the user can also be supported by a large language model (LLM), as known from ChatGPT, for example. The user may interact with such an LLM verbally. For example, a spoken request such as a phrase or question may be received and provided to the LLM. For this purpose, the request may be received by an input device, in particular an input device of the AR device 3, such as a microphone or the like, converted into text by a speech-to-text module and provided to the LLM. However, any other kind of input, such as entering the text by an input terminal, e.g. by means of a keyboard or the like, may be possible, too. The LLM may provide an appropriate response to the input of the user. This response may be provided to the user by an acoustically output, for example a synthetically generated voice output, by providing a text message, providing any other kinds of indication such as lines, arrows, symbols, icons, images and/or video clips. For this purpose, an appropriate output component of the AR device 3 may be used. The output may relate to output which is dynamically generated by the LLM in response to the request of the user. However, the output may also comprise, for example prestored elements which have been generated in advance. For example, the output may comprise prestored images such as images of the medical device 1 in a specific configuration, images of a specific part of the medical device 1, a video clip for illustrating a specific operation of any other kind of data which has been prepared in advance.

When generating the output, the LLM may take into account the language of the user. Accordingly, the LLM may provide the response in the same language as used by the user for formulating the request. For example, when the user asks a question in English, the LLM may also provide the response in English, and when the user asks a question in German, the LLM may provide the response in German. However, it may be also possible to limit the processing and/or the generation of the response to one or more specific languages. Furthermore, it may be also possible to provide a response of the LLM to a specific language regardless of the language in which the input was formulated. For example, the LLM may always provide a response in a language corresponding to which the medical device 1 is set.

The dialogue with the LLM may be used for any kind of interaction, in particular for any kind of assistance which can help the user in operating the medical device 1. For example, the user may ask which component or which part of the medical device 1 is currently located in front of him. For this purpose, it may be also possible to take into account supplementary information. For example, an input component, in particular input component of the AR devices 3, may detect a viewing direction of the user and/or the environment of the user, in particular it may be possible to detect/identify elements or objects in the environment of the user. Accordingly, this information may be also taken into account when the LLM generates a response to the request of the user.

The user may also ask, for example, how to perform a desired operation, for example how to attach an accessory or disposable, or how to execute a next step of the desired operation or the user may ask where a specific element of the medical device is located. In order to generate a response to such a request, it may be possible to take into account any kind of additional information such as information acquired by a sensor component, in particular a sensor component of the AR devices 3. For example, image data of the environment may be captured by an optical sensor component such as a camera. The image data may be processed in order to identify the medical device 1, the configuration of the medical device 1, accessories of the medical device, a spatial relationship between the user and the medical device 1, a current operation of the user or to detect a spatial position of a part of the body of the user, such as the current position of a hand of the user. Further, it may be also possible to receive supplementary information from any other kind of data source. For example, it may be also possible to receive information about the current configuration of the medical device 1, a current state of the medical device 1, operation parameters of the medical device 1 or any other property of the medical device 1 via a wired or wireless communication link.

Accordingly, the user may be assisted by a verbal interaction with the LLM in order to carry out a desired task and/or to get more information about the medical device 1. The information provided by the LLM do not necessarily have to refer only to a task currently being executed by the user. It is also possible for the user to interact with the LLM to obtain any other kind of information, for example general information in connection with the medical device 1.

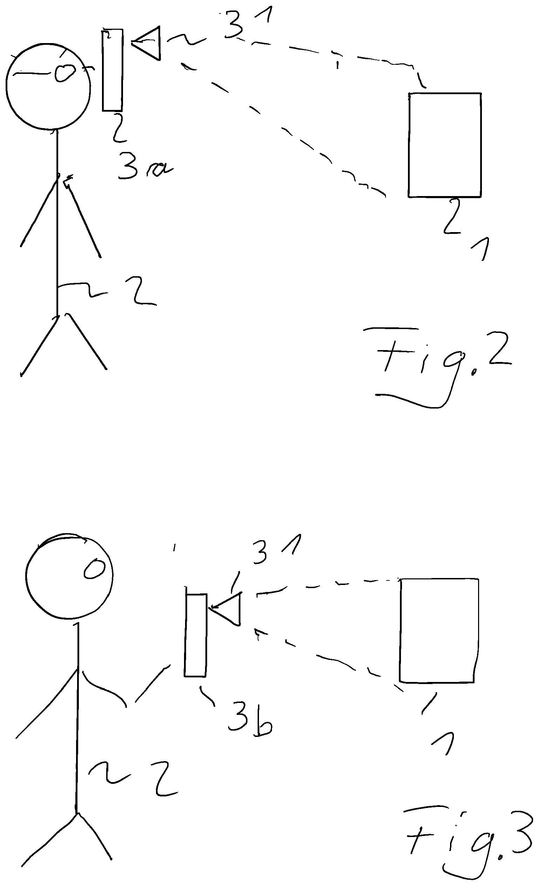

The other components, depicted in Fig., 1 are described below (after Fig. 3) in more detail.

**Figure 2** shows a schematic illustration of an AR device 3a, used when a user handles or operates the medical device 1 or related devices or parts (e.g. disposables), wherein the AR device 3a comprises a headset or AR glasses. In such an embodiment, the AR device 3a may be arranged on a head of the user 2. The user 2 may look through a semi-transparent display.

Accordingly, the user 2 can still recognise the environment. In addition, graphical and/or optical elements may be provided by the semi-transparent display so that the additional graphical and/or optical elements may be shown at positions related to elements in the real world. In addition, the AR device 3a may also comprise further output elements such as loudspeakers or the like in order to provide acoustic output. Further, the AR device 3a may comprise a camera 31 in order to capture image data, in particular image data in a direction in which the user 2 is currently looking. Further, the AR devices 3a may comprise or may be in (in particular wireless) data connection any other kind of sensor components for monitoring a movement of the AR device 3a and/or a corresponding movement of the user 2 or for acquiring any other kind of appropriate sensor data. The AR device 3a may also comprise any other kind of sensors, for example a microphone for receiving voice commands from a user. The sensor component, e.g. in the form of the camera 31 may alternatively or in addition be provided externally to the AR device 3, 3a.

**Figure 3** shows a schematic illustration of another type of AR device 3b. In a possible configuration, the AR device 3b may be realised by a handheld device such as a tablet computer or smartphone. The handheld AR device 3b may comprise a camera 31 for capturing image data, in particular during operation of the medical device 1. Further, the handheld AR device 3b may comprise a display for providing the captured image data in combination with additional graphical elements. In this way, the image data of the real environment may be overlaid with additional optical and/or graphical elements. Alternatively, the handheld AR device 3b may comprise a semi-transparent display so that the user 2 can look through this semi-transparent display. In such a configuration, additional optical and/or graphical elements may be provided on the semi-transparent display in order to provide additional information, in particular information related to one or more elements to which the user 2 is looking through the semi-transparent display. In any case, the handheld AR devices 3b may also comprise output components for providing acoustic and/or haptic output. Further, the handheld AR device 3b may comprise or be connected to any kind of sensor component, e.g. a microphone for sensing acoustic signals, in particular voice command of the user, or sensors for monitoring a movement of the handheld AR device 3b.

The AR device 3, 3a, 3b may be controlled, for example, by means of a control device 4.

In figure 1, this control device 4 is illustrated as a separate component which is arranged external to the medical device 1. However, it may be also possible to implement this control device 4 into the medical device 1 and/or into the AR device 3. Especially, it may be even possible that the functionalities of control device 4 may be realised by components of the medical device 1.

Generally, it is possible and preferred to implement the control device 4 as distributed system. A distributed system may be implemented partially on a first system (e.g., AR device) and partially on a second system (e.g., medical device/dialyses device). It is clear to the person skilled in the art, that also further systems may be used for implementation and/or that the allocation of functions to be executed by the control device 4 (which represent part of the distributed system) may vary.

Control device 4 may receive sensor data from one or more sensor components, in particular sensor components of the AR device 3 or external sensor components, or another device related to AR device 3. Furthermore, control device 4 may also receive further information or a data from the medical device 1. For example, control device 4 may receive data relating to the configuration of the medical device 1, a current setting of the medical device 1, data for specifying a fill level of consumable of the medical device 1, operational data such as, for example, a flow rate, a pressure value or the like, status or error messages and/or any other information which are related to (status and/or operation of) the medical device 1.

Control device 4 may also establish a communication link with one or more external storage devices, in particular one or more databases 5. For example, such a database 5 may provide any kind of information related to the medical device 1. Such information may comprise, for example, any kind of data for specifying the medical device 1. For instance, control device 4 may receive a serial number of the medical device 1 and refer to a database 5 in order to obtain further information in connection with this serial number. It may be also possible to use image data, in particular image data captured by a sensor component of the AR device 3 in order to identify the medical device 1 or an attachment/accessory of the medical device 1. For example, a QR code or barcode may be scanned and control device 4 may refer to database 5 in order to identify the medical device 1 based on this code. Alternatively, it may be also possible to identify any appropriate features in an image representing the medical device 1, and to refer to a database 5 in order to identify the medical device 1 based on these features. However, any other approach for identifying a medical device 1, an accessory or attachment, or to retrieve any other information in connection with the medical device 1 may be possible, too.

Database 5 may also provide, for example, further relevant information such as recommended settings, a fill level of consumables, predefined operations for a user for operating the medical device 1 of any other kind of appropriate information in connection with medical device 1.

Database 5 may also provide any kind of information for specifying operations in order to carry out a specific task in connection with the medical device 1. Such a specific task may be, for example, a task for configuring the medical device 1, e.g. during a setup procedure, a task for operating or monitoring the medical device 1 during operation, a task in order to resolve a warning or failure of the medical device 1, a task for maintenance of the medical device 1 or even a task for repairing the medical device 1. However, any other kind of task may be specified, too.

Database 5 may also comprise further information in connection with one or more users 2. For example, the database 5 may store and provide any kind of preferences of a user 2. Such preferences may comprise, for example, a specific configuration, in particular a specific configuration for providing information by the AR devices 3, e.g. a type of the representation of the virtual character or person (e.g, which type of avatar or personalized avatar), a colour scheme, a specification how detailed a support/assistance for the respective user 2 should be, a degree of knowledge or a skill level of the respective user 2, language skills, physical limitations of the user 2, or any other data in connection with the user 2.

Further, databases 5 may also provide any kind of information in connection with a patient which shall be treated by the medical device 1. Such information may comprise, for example, a physical characterisation of the patient, a desired therapy/treatment, medication of the patient or any other relevant data in connection with the patient.

In addition, database 5 may also provide any other kind of relevant information which could be useful in connection with an operation of the medical device 1.

Even though database 5 is illustrated in figure 1 as a single database 5, it is understood, that database 5 may be also realised by multiple separate databases. For example, separate databases may be provided for the properties of the medical device 1, data in connection with the operation of the medical device 1, the data in connection with a patient, the preferences of the user, etc. The individual databases 5 may be also located at different spatial locations. Further to this, it may be also possible to store at least some of the data in a local database within the control device 4. In a preferred embodiment, the database is a relational database. In another preferred embodiment, the database is an object-oriented database.

In the medical system as illustrated, for example, in figure 1, the user 2 may be provided with information by the AR device 3. This information may comprise any kind of appropriate information which could be useful for the user 2 when operating the medical device 1. Beyond numerous different manners of providing such information, information may be provided to a user, for example, by a virtual companion such as a virtual character or person. For instance, the virtual representation may be in the form of a virtual companion, which may be represented by a static or dynamic avatar.

The generated output with information for assisting the user may be provided, for example, in a one-way manner, wherein only messages with information for a specific context, such as carrying out a specific task, are provided to the user. Thus, messages in form of the generated output are provided on the AR device. Alternatively, the generated output with information for assisting the user may be also provided in a two-way manner, especially in form of a message exchange (representing a dialogue between the user 2 and the virtual person or character) between the input component (e.g., a user interface, which may be implemented on the medical device 1 or on the AR device or on a separate, e.g., mobile, device of the system) and the output component of the AR device. In the second case, a response to input, activities or questions from the user may be computed and provided to the user 2. Here, messages are sent via a user input device (or input component) to the control device 4.

For providing such assistance to the user 2 in form of a virtual character or person, the AR device 3 may be used. In the following, an approach for controlling an AR devices 3 is described which may perform a computer implemented generation of a virtual character or person on an AR device for assisting the user 2 operating the medical devices 1.

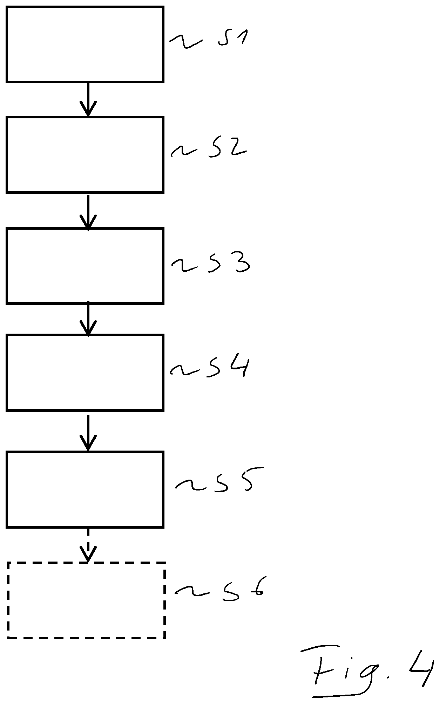

**Figure 4** shows a flow diagram for controlling an AR devices 3. The AR device 3 may be used for assisting a user 2 on or at a medical device 1 according to an embodiment.

The term "on or at" refers to the fact that the assistance method may be used, when or during working with the medical device. All tasks or functions and/or actions to be performed in this respect may be supported. It is not necessarily required that the user directly operates on the medical device but may e.g. work at accessory devices, like e.g., disposables or related devices in the context of the (intended) treatment with the medical device. Further, assistance is also available in a setup or configuration phase of the medical device before the actual operation of the medical device.

Generally, the method may be implemented, for example, in the above-described medical system. Thus, the embodiments relating to systems, devices or apparatuses may comprise any kind of device, component or unit for implementing the features of the embodiments relating to the method as described above. Accordingly, embodiments relating to the method may comprise any appropriate method steps for realising a feature as described in connection with an embodiment relating to a system, apparatus or device.

The method for controlling the AR device 3 may comprise an optional step S1 for receiving input data, in particular input data from the medical device 1. The input data may be received, for example, by means of an input interface which is communicatively coupled with the medical device 1. In this way, any kind of appropriate data may be obtained from the medical device 1. For example, the data may comprise any kind of data for specifying the medical device such as a serial number, a specification for a specific type of the medical device 1, information specifying a current configuration of the medical device 1, information specifying a status of the medical device 1, error messages or any other appropriate information in connection with the medical device 1.

Additionally or alternatively, it may be also possible to receive input data related to the medical device 1 of by means of a sensor component, in particular a sensor component of the AR devices 3. The sensor component may be, for example, an image capturing device such as a camera. For example, such an image capturing device may capture image data in a direction on which the user 2 is currently looking or on which the AR devices 3 is directed to. The data may be also captured, for example, by means of a microphone or the like for acquiring acoustic signals. For example, a sensor component may receive voice commands from the user 2. Further, a sensor component may read a QR code, a barcode, and RFID tag or the like. Further to this, a sensor component may be used for detecting a position and/or a movement of the user. For this purpose, appropriate sensors such as a gyroscope, an acceleration sensor or the like may be used. Furthermore, data in connection with the medical device 1 may also be received, for example, by an appropriate interface such as a wired or wireless communication channel.

In a further step S2, the method computes an assistance scheme for the user. This assistance scheme may comprise, for example, a scheme for determining the manner(s) (e.g., how, in which form, when how long etc.) how the output to be generated should be provided. The output serves for assisting in carrying out a task to be executed by the user. The assistance scheme may be computed based on the received input data from the dialysis device. The assistance scheme may be computed, for example, by applying artificial intelligence module or a machine learning approach. For example, a neural network may be used which is provided with appropriate information such as information about the medical device 1, the task to be executed, preferences or constraints related to the user 2 and/or other relevant information. As a result, the neural network may output data of the assistance scheme, which are individually computed for the present demands.

The assistance scheme may comprise any kind of appropriate data, rules, specifications or the like which are useful for providing the user with an assistance to carry out the desired task on the medical device 1. In particular, the assistance scheme may comprise appropriate data for determining generation of a virtual person or character which can be provided to the user 2 by the AR device 3.

Accordingly, in a step S3 output on an output component of the AR device 3 is generated. The output on the output component of the AR device 3 is generated by applying the computed assistance scheme. Especially, the generated output may comprise a representation of a virtual character or person. This representation of the virtual character a person may comprise optical, acoustic or and/or haptic output. In this way, the user is provided on the AR devices 3 with a virtual character person which can guide the user to perform the desired task on the medical device 1. Such a guidance by the artificially generated virtual character or person may come very close to an assistance of a real person. Thus, such an assistance may be experienced to be very comfortable by the user 2.

The virtual person or character may be generated in a variety of ways. For example, the virtual person or character may be a static image or representation. For instance, a virtual person may be provided by an image (e.g. photo) of a real person or a computer-generated artificial image of a person. Furthermore, any other appropriate static image of a character or object may be possible, too. For example, the person or character may point to a specific position on the medical device 1 in order to indicate a desired operation. Additional optical information may be provided, for example, by further graphical elements such as lines, arrows, icons of the like. Further, it may be also possible to provide text messages in a text box, balloon or the like.

As an alternative to static representations, it is also possible to generate a dynamic virtual person or character. Such a dynamic virtual personal character may visualise the course of an action or operation to be performed by the user. For example, a dynamic virtual person may be a (computer-generated or computer animated) representation of a person which simulates a desired operation on the medical device 1. Alternatively, it may be also possible to generate only a representation of a part of the complete person, for example only an arm with a hand or only a hand with fingers. In this way, only a small part of the medical device 1 may be covered by the virtual person or character.

Alternatively or in addition, it is possible to switch between the static and dynamic representation. The switch may be triggered on a user interface, by activating a switch button. Alternatively or in addition, the switch may be pre-configured according to pre-definable rules, which may be stored in a rules database. The rules e.g. may specify that very important tasks or very complicated tasks may be assisted with a dynamic representation and/or less important tasks or easy-to-execute tasks may be assisted with a static representation. In a configuration phase it may be configured, which tasks are classified as important or less important or complicated or easy-to-execute. In this configuration phase, also the rules may be defined.

Beyond a (computer-generated) representation of a realistic person, the virtual character may be also an artificial object, for example a cartoon character, a star, or any other artificial representation.

In order to generate an appropriate representation of the virtual person or character, representational data may be processed, for example, information, rules and/or algorithms of limb actions which shall be performed by the virtual person or character. For example, these data may specify how the fingers of a virtual hand shall move when providing the virtual person or character demonstrating an operation on the medical device 1. However, actions or movements of any other parts of the body of the virtual person or character may be also specified in the computed assistance scheme. In this way, the computer implemented generation of the virtual person or character can be performed in a very efficient and realistic manner.

Accordingly, the specifications of the computed assistance scheme serve as a basis for generating the static or dynamic representation of the virtual person or character.

Further to the optic representation of the virtual person or character, is also possible to provide acoustic and/or haptic output related to a virtual person or character. For example, the virtual person or character may be represented by generating sound signals which may be output by an output component of the AR device 3. The sound signals may comprise, for instance, spoken text for instructing the user 2 and/or explaining the medical device 1. However, the sound signals may also comprise a warning tone, a melody or any other acoustic signal. Further, haptic output such as a shaking or vibration signal may be also provided to the user 2.

The appearance of the virtual character or person which is generated in step S3 may be adjusted. In particular, the appearance of the virtual person or character may be adjusted depending on the preferences and/or demands of the user 2. The preferences and/or demands of the user 2 may be represented by one or more properties of the virtual person or character. For example, the user may specify colour of hair, skin, clothes or the like, gender (male or female or diverse), properties of a voice, and/or any other appropriate property which may be adjustable for the virtual person or character. For example, the user may enter one or more properties by means of an appropriate input device such as a user interface or the like. In a possible embodiment, the user may select a virtual character or person out of a number of predefined characters or persons. In this case, the data of the predefined characters or persons may be stored in an appropriate local or remote memory. Further, it may be also possible to store the selection of the user and to retrieve the stored selection at a later point in time again.

The user 2 may be provided with assistance to carry out a task to be executed by the output of the virtual person or character which is provided by the output component of the AR devices 3. During the operation of the medical device 1, an inexperienced user 2 may need more help than an experienced user 2. Hence, it is desirable to provide the inexperienced user 2 with a sufficiently detailed assistance so that the inexperienced user 2 is enabled to carry out the desired task reliably and correctly. For this, the inexperienced user 2 might be provided with an assistance having a relative high level of detail. However, such a high level of detail may be annoying to an experienced user. Accordingly, it may be possible to adapt the level of detail depending on the needs of the user 2.

In this connection, the level of detail may be understood as an amount of information which is provided by the virtual person or character. For example, the level of detail may relate to a number of intermediate steps or sub-tasks which are provided to the user 2. For example, a high level of detail may comprise a high number of intermediate steps or sub-task which are provided to the user, while at least some of the intermediate steps or sub-task may be omitted for a lower level of detail. Depending on the desired graduation, multiple levels of detail may be possible with a graduated reduction of information from higher levels of detail to the lower levels of detail.

Further, the level of detail may also relate to an amount of information which is provided for each task, sub-task or step. For example, a higher level of detail may comprise more information which results in a longer text or a longer voice output or a more detailed representation of the generated virtual person or character. A lower level of detail may comprise only a reduced amount information which may result in a shorter text or a shorter voice output or a less detailed representation of the generated virtual person or character. Further, depending on a level of detail, more or less additional indications such as lines, arrows, icons or the like may be provided in association with the virtual person or character.

It may be also possible to use a highly animated virtual person or character for a higher level of detail, wherein the degree of animation is reduced with lower levels of detail. Further, it could be also possible to use a more realistic virtual person or character for a higher level of detail and a less realistic virtual person for a lower level of detail. However, it is understood, that any other appropriate scheme for adapting the level of detail may be possible, too.

In one possible implementation, a user may manually specify or select a level of detail, which should be applied to the generated virtual person or character.

Additionally or alternatively, the level of detail may be also determined in an automated manner. For example, it may be possible to monitored the activities of the user 2. Based on the monitored activities, it may be possible to evaluate a skill level of the user 2. For example, the activities of the user 2 may be compared with corresponding reference operations. The reference operations may be provided, for example by a local or remote storage device. Depending on a matching between the activities of the user 2 and the corresponding reference operations, a skill level may be evaluated. Furthermore, it may be also possible to evaluate a period of time which was necessary for the user to carry out a specific task or operation (representing: how long does it take to execute the task?). Alternatively or in addition it is possible to evaluate historical data. The historical data represent how often the particular user has operated the medical device, e.g., and a specific type of device in the past (representing how experienced the user is for the device or the type of device). However, any other approach for determining the skill level of the user 2 may be possible.

Based on the determined skill level of the user 2, a related level of detail and/or assistance scheme may be selected. For example, a reference table for assigning a level of detail to the determined skill level of the user 2 may be provided. However, any other appropriate scheme for matching a skill level of the user 2 and the corresponding level of detail may be possible, too.

Further, it may be also possible to obtain a skill level of the user 2 from another data source, for example, a local or remote storage device. For example, previous operations of the user 2 may be recorded and/or evaluated, and a skill level of the user 2 may be determined on these previous operations of the user 2. Further, it may be also possible to consider previous training sessions or any other information which may be useful in order to assess skill level of the user 2. Accordingly, the level of detail for the virtual person or character may be adapted based on this skill level, which may in particular comprise an experience level obtained from historical data.

Additionally or alternatively, preferences of the user with respect to the level of detail and/or any other preferences with respect to the virtual person or character may be stored, for example in a local or remote database, and retrieved in order to adapt the level of detail or the generation of the virtual person or character according to the stored preferences.

Further to this, any kind of user input from the user 2 may be taken into account for computing the assistance scheme and/or generating the virtual person or character. For this purpose, the user 2 may enter information by means of a user interface or input component. Alternatively of in addition, it may be also possible to use a sensor component, e.g., a sensor component of the AR device 3, in order to monitor the user, his activities and identify a user input based on the monitoring results of the user 2. For example, a microphone (e.g. of the AR device 3) may be used in order to receive voice commands. The voice commands may be used in order to set up or adjust the operation for generating the assistance scheme and/or the generation of the virtual person or character. Furthermore, it may be also possible to analyse the voice of the user 2 in order to establish a dialogue between the user 2 and the virtual person or character.

Additionally or alternatively, it may be also possible to monitor the user 2 in order to detect specific gestures or motion patterns. For example, one or more gesture(s) or motion pattern(s) may be associated with corresponding commands.

Based on the detected gestures, motion patterns, voice commands, haptic input or any other activity of the user 2, a related operation for controlling the AR device 3, in particular for providing assistance by the virtual character person may be triggered. For example, the provision of assistance by the virtual person or character may be switched on or off based on such a command. Further, a level of detail may be adapted, additional information may be provided or any other appropriate operation may be started, stopped or adapted.

In order to further simplify and automate the configuration, the assistance scheme and/or the generation of the virtual corrector or a person may be also adapted based on further parameters. For example, a user may be automatically identified by the AR devices 3 and the configuration for providing the assistance may be adapted depending on previously stored preferences of the identified user 2. Further, it may be also possible to acquire regional information such as a country where the AR devices 3 and the medical device 1 are currently operated. Accordingly, a language for providing text or speech may be automatically adapted based on the identified regional properties. However, such language settings may be overridden by user preferences.

Further to this, it may be also possible to take into account any other kind of appropriate information, such as time information, information about the premises where the AR device 3 and the medical device 1 are currently located, or the like. For example, the assistance scheme and/or the virtual person or character may be adapted depending on whether the medical device 1 is operated in a hospital or at a patient's home.

Furthermore, it may be also possible to take into account additional constraints, such as an environment of the medical device 1. For example, a sensor component of the AR devices 3 may scan the environment of the medical device 1 in order to determine an available space surrounding the medical device 1. Accordingly, the computation of the assistance scheme and/or the generation for the virtual person or character may take into account the spatial constraints. For example, the virtual person or character may be provided so that the person or character is not in conflict with another object in the surrounding of the medical device 1.

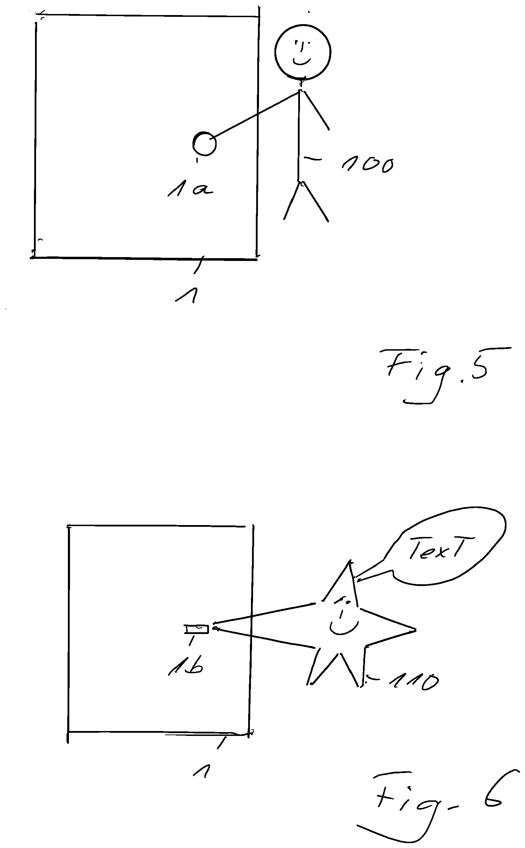

**Figure 5** shows a schematic illustration of a possible representation for an assistance of the user 2 by a virtual person 100. Even though the person 100 is only illustrated very schematically, it may be possible to increase the degree of reliability as high if desired. Instead of a whole virtual person 100, it may be also possible to provide only a part of the person, for example only and armed with a hand, or only a hand with fingers. The virtual person 100 may be a static or dynamic representation. In case the presentation is a dynamic representation, the virtual person 100 may demonstrate a virtual operation on a control element 1a of the medical device 1.lt may be possible to enrich the demonstration with further information such as spoken text, or the like.

**Figure 6** shows a schematic illustration of a further possible presentation for an output generation with assistance for the user by means of a virtual character 110. The virtual character 110 may be also a static or dynamic virtual representation. As can be seen in figure 6, even such a virtual character can be used to indicate a desired operation on a control element 1b of the medical device 1.

As further demonstrated in figure 6, additional information may be provided, for example, by a text bubble 111 in association with the virtual character 100.

Summarising, the present invention relates to a system for an automated generation and provision of assistance when operating a medical device such as a dialysis device. In particular, the present invention provides an automated computation of a virtual assistant in form of a representation of a person or character, which may be actuated. The virtual assistant may be provided on an output component, e.g. by means of an augmented reality device.

## Claims

1. Method for controlling an augmented reality, AR, device (3), wherein the AR device (3) is used for operating a dialysis device (1), the method comprising:
- computing (S2) an assistance scheme for carrying out a task to be executed by the user (2) with regard to the dialysis device (1);
- generating (S3) an output on an output component of the AR device (3), wherein the output on the output component of the AR device (3) is generated by applying the computed assistance scheme, and
wherein the generated output comprises a representation of a virtual character or person.

2. Method according to any of claims 1, wherein the assistance scheme and/or the output is computed by applying a machine learning tool and/or
wherein the method further comprises receiving (S1) input data from the dialysis device (1), and wherein computing the assistance scheme is based on the received input data from the dialysis device (1);

3. Method according to claim 1 or 2, wherein the generated output comprises optical, acoustic and/or haptic output on the output component of the AR device (3).

4. Method according to any of preceding claims 1 to 3, wherein the computed assistance scheme comprises rules for representation of limb actuations of the virtual character or person and/or wherein in the generated output the limbs of the virtual character or person are actuated.

5. Method according to claim 4, wherein the limb actuations of the virtual character or person represent or indicate a movement that is required to operate the dialysis device (1).

6. Method according to any of preceding claims 1 to 5, wherein a level of detail for generating the output is adjustable.

7. Method according to claim 6, comprising monitoring activities of the user for determining a skill level of the user (2), and/or
wherein the level of detail for generating the output is adjusted based on the determined skill level of the user (2).

8. Method according to claim 6 or 7, comprising receiving data related to a skill level of the user (2) and/or preferences of the user (2) from a database, and/or
wherein the level of detail for generating the output is adjusted based on the received skill level and/or preferences of the user (2).

9. Method according to any of preceding claims 1 to 8, wherein the assistance scheme and/or the output is computed based on information received from the AR device (3), in particular based on a type of dialysis device (1), monitored activities of the user (2), a user identity, a geographical region identifier and/or a time information.

10. Method according to any of the preceding claims 1 to 9, comprising receiving user input by an input component, in particular wherein the user input comprises a gesture, a voice input and/or a haptic input, and/or
wherein the assistance scheme and/or the generated output is computed based on the received user input.

11. Method according to any of the preceding claims 1 to 10, comprising:
scanning a spatial environment of the dialysis device (1) by at least one sensor component,
wherein the assistance scheme and/or the generated output is computed based on data of the scanned spatial environment.

12. Method according to any of preceding claims 1 to 11, wherein the representation of a virtual character or person is adjustable by the user (1), in particular, wherein the representation of a virtual character or person is selectable out of a number of prestored templates.

13. Augmented Reality, AR, system for use in a medical environment comprising a dialysis device (1), the AR system comprising:
an AR device (3); and
a control device (4), configured to execute the method according to any of the claims 1 to 12 and to:
- compute an assistance scheme for carrying out a task to be executed by the user (2);
- generate an output on an output component of the AR device (3),
wherein the output on the output component of the AR device (3) is generated by applying the computed assistance scheme, and
wherein the generated output comprises a representation of a virtual character or person.

14. AR system according to claim 13, comprising or in data connection with a sensor component configured to capture data related with the dialysis device (1) and/or data related with an operation of a user (2).

15. Medical system, comprising:
a dialysis device (1); and
an AR system according to claim 13 or 14.
